(19) **Europäisches Patentamt / European Patent Office / Office européen des brevets**

(11) **EP 3 257 503 A1**

(12) **EUROPEAN PATENT APPLICATION**
published in accordance with Art. 153(4) EPC

(43) Date of publication:
**20.12.2017 Bulletin 2017/51**

(21) Application number: **16748762.8**

(22) Date of filing: **08.02.2016**

(51) Int Cl.:
**A61K 9/52** (2006.01)      **B82Y 5/00** (2011.01)

(86) International application number:
**PCT/ES2016/000016**

(87) International publication number:
**WO 2016/128591 (18.08.2016 Gazette 2016/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **09.02.2015 ES 201500129**

(71) Applicants:
• **Universidad De Sevilla**
 **41013 Sevilla (ES)**
• **Universidad de Cádiz**
 **11003 Cádiz (ES)**

• **Centro de Investigación Biomédica en Red (CIBER)**
 **28029 Madrid (ES)**

(72) Inventors:
• **MARTÍN BANDERAS, Lucía**
 **41012 Sevilla (ES)**
• **FERNÁNDEZ ARÉVALO, Mercedes**
 **41012 Sevilla (ES)**
• **BERROCOSO DOMINGUEZ, Esther**
 **11510 Puerto Real (Cádiz) (ES)**
• **MICO SEGURA, Juan Antonio**
 **11003 Cádiz (ES)**

(74) Representative: **Pons**
 **Glorieta Ruben Dario 4**
 **28010 Madrid (ES)**

(54) **METHOD FOR PRODUCING A PHARMACEUTICAL COMPOSITION OF POLYMERIC NANOPARTICLES FOR TREATING NEUROPATHIC PAIN CAUSED BY PERIPHERAL NERVE COMPRESSION**

(57)      The object of the present invention is the method for obtaining a pharmaceutical composition of polymeric nanoparticles for the treatment of neuropathic pain caused by peripheral nerve compression, related to the pharmaceutical industry and nanotechnology. The invention particularly relates to a novel system for orally administering the cannabinoid drug CB13 based on polymeric nanoparticles capable of alleviating pain for nine days following a single oral administration of an aqueous suspension of nanoparticles.

**Description**

**Object of the Invention**

The object of the present invention relates to a novel system for orally administering the cannabinoid drug CB13 based on polymeric nanoparticles capable of alleviating neuropathic pain caused by peripheral nerve compression in rats for nine days following a single oral administration of an aqueous suspension of nanoparticles.

This neuropathic pain model can be equivalent, given the proper adaptation of a pre-clinical model to the disorder in humans, to a post-traumatic, post-surgical or nerve entrapment neuropathic pain model. In general, this model is recognized as a mechanical-type neuropathic pain model in order to distinguish it from a model that represents peripheral neuropathic pain of a metabolic or toxic (chemical or viral) type, or that caused by neurodegenerative diseases.

Current drug treatment for chronic pain is not effective in many clinical situations. Specifically, neuropathic pain caused by peripheral nerve compression is one of the unmet needs in chronic pain management.

Recently discovered cannabinoid drugs have proven to be helpful in treating some types of neuropathic pain. However, due to their high lipophilicity and given they are not readily available to be taken orally, designing novel systems for oral administration, the physiologically most accepted way of administering drugs, is necessary.

The present invention relates to the pharmaceutical industry and nanotechnology, and it particularly relates to a novel system for orally administering cannabinoids for the treatment of neuropathic pain caused by peripheral nerve compression.

The final composition combines the cannabinoid drug CB13, the design of a nanoparticle based on the poly(lactic-co-glycolic acid) copolymer (PLGA), an active material on the particle surface, as well as a cryoprotectant.

The system, administered in a single oral dose, allows for the effective treatment of neuropathic pain caused by peripheral nerve compression in rats for nine days.

**Neuropathic pain**

Neuropathic pain is defined as the pain "arising as a direct consequence of a lesion or disease affecting the somatosensory system" (Haanpää et al., 2010).

Neuropathic pain can be the result of a lesion that affects the peripheral as well as central nervous system.

Currently, neuropathic pain is defined as "the pain initiated or caused by a primary lesion or dysfunction in the nervous system".

It is understood that the expression "nervous system" is replaced by "somatosensory system" to distinguish neuropathic pain from the pain caused by lesions to other parts of the nervous system, for example, pain associated with muscle spasticity in multiple sclerosis.

The term Neuropathic Pain includes any type of lesion or primary dysfunction that affects the peripheral nervous system as well as the central nervous system or the sympathetic system. Thus, Neuropathic Pain can be classified into three major categories:

a) Peripheral Neuropathic Pain: changes originate in the Peripheral Nervous System, such as neuropathic pain caused by peripheral nerve compression (object of this invention).
b) Central Neuropathic Pain: the lesion or primary dysfunction is located in the Central Nervous System.
c) Sympathetic Pain: the origin of which is in the Sympathetic Nervous System.

**State of the art**

*Treatment of neuropathic pain*

Drug combinations are frequently used in the routine clinical treatment of neuropathic pain, the drug treatments used being the following */**:

(*) Some are not indicated in the Technical Data Sheet. (**) Those used to treat peripheral neuropathic pain, which is most common

- Tricyclic antidepressants:

  - Amitriptyline*: indicated (only) for chronic pain.
  - Nortriptyline*: not indicated for the treatment of neuropathic pain.

- Other antidepressants:

  - Duloxetine**: only indicated for the treatment of diabetic peripheral neuropathic pain in adults.
  - Venlafaxine*: not indicated for the treatment of neuropathic pain.
  - Desvenlafaxine*: not indicated for the treatment of neuropathic pain.

- Anticonvulsants:

  - Gabapentin**: indicated for the treatment of peripheral neuropathic pain in adults.
  - Pregabalin**: indicated for the treatment of peripheral and central neuropathic pain in adults.

- Topical treatments:

  - Capsaicin cream 0.075%**: indicated for the relief of moderate to severe pain in painful diabetic neuropathy, which interferes with daily activities and has not responded to other treatments.
  - Lidocaine patch 5%**: indicated for the symptomatic relief of neuropathic pain associated with a previous herpes zoster infection (postherpetic neuralgia).
  - Capsaicin patch 8%**: indicated for the treatment of peripheral neuropathic pain in non-diabetic adults.

- Opioids (drugs used as a last resort and normally associated with any of the aforementioned drugs):

  - Tramadol, oxycodone, morphine, fentanyl, tapentadol and buprenorphine.

*Cannabinoids for the treatment of neuropathic pain*

Cannabinoids make up a group of psychoactive compounds present in the resin secreted from the leaves and buds of the Cannabis Sativa plant. The plant contains approximately 400 different chemical compounds, among which about 60 are considered to be part of the group of cannabinoids (Nocerino et al., 2000).

$\Delta^9$-tetrahydrocannabinol ($\Delta^9$-THC) is the most abundant compound and the main cannabinoid responsible for the psychoactive properties. The administration of cannabinoid compounds is also associated with the emergence of the following effects: analgesic, antiemetic, cardiovascular, neuroendocrine, immunomodulatory and antiproliferative effects, and actions on muscular activity, among others. Thanks to the broad therapeutic spectrum they present, authorities as well as the scientific community consider cannabinoids to be a powerful therapeutic group that should be explored.

Among the drug effects produced by cannabinoids, antinociception is one of the most notable therapeutic applications. The administration of these compounds could provide a response to important unmet medical needs, such as certain chronic pain illnesses that do not respond to current therapy based on anti-inflammatory drugs and/or opioid drugs. In this sense and with the aim of reducing the psychoactive effects, thanks to the discovery of new receptors and advances in medical chemistry, new and powerful cannabinoids have been synthesized.

Studies have focused on the design of a new class of ligand agonists of cannabinoid receptors, known as aminoalkylindoles, which have shown good activity in CB1 and CB2 receptors in humans. This led to the discovery of a new CB1/CB2 dual agonist, 1-naphthalenyl[4-(pentyloxy)-1-naphthalenyl]methanone (CB13, Novartis), which shows powerful antihyperalgesic activity in animal models and limited penetration into the brain (Dziadulewicz et al., 2007).

In behavioral studies of chronic (neuropathic and nociceptive) pain, CB13 reverses the established mechanical hyperalgesia after oral administration and local injection into the hind paw of the animal models (pig and rat). In behavioral tests on the activity of the central nervous system in rats, CB13 had significant effects at a dose only 20 times greater than the oral dose required to reverse hyperalgesia. Therefore, this data indicates that CB13 produces antihyperalgesic activity predominantly through an action on the peripheral sensory nerves (Gardin et al., 2009).

The administration of these drugs and, more specifically, oral administration is currently a major challenge. This is fundamentally due to: a highly lipophilic nature, marked effect of the first step, physiochemical instability and various side effects.

CB13 belongs to the class II (low solubility and high permeability) components of the Biopharmaceutics Classification System (BCS), having very low aqueous solubility (-0.001-0.002 mg/mL). As a result of its low solubility and dissolution in gastrointestinal fluids, CB13 is not completely absorbed after oral administration (Gardin et al., 2009).

*Polymeric nanoparticles as prolonged drug delivery systems*

In recent years, nanotechnology has evolved in an unprecedented way, reaching a unique state in which its growing progress can neither be foreseen nor prevented. This growth has been observed in several areas, playing a significant role in the area of medicine, offering important advances in: the treatment of diseases, analytical and diagnostic techniques and regenerative medicine.

Pharmaceutical nanotechnology and, more specifically, polymeric nanoparticles currently offer many advantages over "traditional" drug administration systems. The main reason is due to their surface / mass ratio, which is much greater when compared to other systems. This leads to a greater capacity to transport therapeutic molecules.

Prolonged drug delivery through biodegradable and biocompatible polymeric nanoparticles is creating a strong effect on the development of new drug forms with very important economic implications. It was estimated that the global drug delivery market would reach a turnover of €200 billion in 2017 (http://www.marketsandmarkets.com/Market-Reports/drug-delivery-technologies-market-1085.html). Currently, around 75% of new drugs approved by the FDA are improved formulations of known drugs (Bossart et al., 2014).

For an oral administration system based on nanoparticles to be successful, certain basic requirements must be fulfilled, such as low toxicity, optimal properties for transporting and delivering the drug, and a long half-life (Boisseau et al., 2014).

There are many types of drug delivery systems, which include: micelles, liposomes, dendrimers, nanoparticles, nanotubes and polymeric conjugates. They differ in their composition and structure, but they all have the same purpose.

Among prolonged drug delivery systems, the use of polymeric nanoparticles is highlighted, which can remain in the organism during an extended period of time, thus ensuring correct drug delivery. One of the most outstanding polymers is the poly(lactic-co-glycolic acid) copolymer (PLGA) due to its high biocompatibility, low toxicity and high control of drug delivery. Other polymers of interest are: gelatins, dextrans, chitosans, lipids, phospholipids, polycyanoacrylates, polyesters, poly($\varepsilon$-caprolactone) (PCL) (Hudson and Margaritis, 2014; Lai et al., 2014; Lam and Gambari, 2014).

However, these polymers have certain disadvantages that make them less appealing when designing a prolonged oral delivery system. The composition of gelatins and chitosans may exhibit a certain degree of heterogeneity, which can lead to variability during the synthesis of particles. On the other hand, the chemical process for forming the particle can give rise to a certain degree of immunogenicity to the particle (Wacker, 2014).

Although polyesters have been widely used to control drug delivery, they are mainly used in cosmetics.

Poly(alkyl cyanoacrylates) (PACAs) have been approved by the FDA (ethyl, n-butyl and octyl cyanoacrylate) for parenteral administration. In 2005, the FDA approved a formulation of doxorubicin and polyiso-hexyl-cyanoacrylate nanoparticles for the treatment of primary hepatocellular carcinoma, carrying out clinical trials in Phase III (Transdrug®, BioAlliance Pharma, France).

With the correct selection of the polymer and a suitable design, these systems can: diminish adverse effects, control drug delivery, extend the drug's therapeutic effect over time, reduce the number of doses per day, reduce treatment costs, reduce toxicity and provide greater specificity against target tissue.

For this, basic aspects such as size and distribution of sizes or surface properties must be very carefully controlled. Thus, surface modification and/or functionalization with polymer molecules such as polyethylene glycol (PEG, PEGylation) or copolymers such as poloxamers have proven to be useful. In particular, PEGylation is of special interest after parenteral administration since it has shown to prevent the opsonization of nanoparticles by the reticulo-endothelial system, thus prolonging residence time in the bloodstream (Owens and Peppas, 2006). After oral administration of these PEGylated systems, it was verified that greater stability is obtained since degradation by digestive enzymes as well as pH is reduced (Vila et al., 2002; Jain et al., 2010).

Given the foregoing, the present invention consists of covalently bonded polyethylene glycol (PEG) and biodegradable and biocompatible poly(lactic-co-glycolic acid) copolymer (PLGA) nanoparticles and the drug CB13 to generate a new pharmaceutical system of oral administration that makes it possible for neuropathic pain caused by peripheral nerve compression to recede for days after a single dose.

Currently, there are four cannabinoid-based drugs marketed for the treatment of different illnesses:

- Sativex® (tetrahydrocannabinol + cannabidiol), a sublingual spray for the treatment of spasticity in multiple sclerosis.
- Cesamet® (nabilone, synthetic derivative).
- Marinol® (dronabinol, synthetic derivative) as an antiemetic in chemotherapy.
- Namisol® in Clinical Phase II for the treatment of neurodegenerative diseases, pain and multiple sclerosis.

A document in The International Journal of Nanomedicine 2012: 7 5793-5806 by Martin-Banderas et al. describes the synthesis of PLGA biocompatible and biodegradable nanoparticles and CB13 for possible oral administration. PLGA particles with a homogeneous size of 90-300 nm were not cytotoxic. The specific use of PLGA-PEG with CB13 and its in vivo effectiveness are not described in this document.

A document in Mol. Pharm. 2013, 10, 4546-4551 by Shen et al. describes the use of curcumin-loaded PLGA nanoparticles administered orally to mice with a tolerance to opioids. PLGA, PEGylated or partially PEGylated nanoparticles had diameters in the range of 155-166 nm and were obtained through a novel multiple mixing method (MIVM).

The degree of antinociception was assessed by using the hot plate test and the tail-flick test. It was found that the non-PEGylated PLGA nanoparticles offered a higher level of restoration of antinociception for morphine. The authors do not describe the duration of the effect after oral administration.

A document in Drug Dev.Ind.Pharm., 2013, 39, 6, 854-864 by Lalani et al. describes the use of tramadol-loaded PLGA nanoparticles and a surface modified with transferrin or lactoferrin in order to pass through the blood-brain barrier. The nanoparticles were obtained through nanoprecipitation and had diameters smaller than 150 nm. The nanoparticles modified with lactoferrin had a greater antinociceptive effect than those modified with transferrin or those that were not modified. The authors do not describe the use of PEG.

A document in Neuromodulation, 2012;15(6):520-6 by Milligan et al. describes the use of PLGA microparticles loaded with pDNA IL-10 gene in an animal model of neuropathic pain. The particles are administered intrathecally. In this way, the particles are quickly phagocytized, favoring gene transfection and IL-10 gene expression for 8 weeks.

A document in Eur J Pharm Biopharm, 2009; 72(1):54-61 by Ratajczak-Enselme M et al. describes the use of PLGA microparticles loaded with the ropivacaine anesthetic for epidural (local) administration to treat post-operative pain in a sheep animal model. The concentration of ropivacaine in the CSF after epidural administration was sustained over time, and its systematic absorption was lower, reducing toxicity and favoring its entry into the meninges.

A document in Spine 2008; 33(3), 227-234, by Zanella et al. describes the use of a formulation based on PLGA for administrating the alpha tumor necrosis factor (TNF$\alpha$), which induces mechanical allodynia and hyperalgesia. The polymeric formulation consists of 1 cm long cylinders that were administered locally (ipsilateral or contralateral) in an animal model with chronic pain caused by sciatic nerve constriction (CCI). Mechanical allodynia was assessed by using the von Frey test and the Hargreveas test was used for thermal hyperalgesia. The designed formulation, in the form of a polymeric deposit, provided analgesia over a period of 59 days.

A document in Arthritis Research & Therapy, 2012, 14, 4, R179 by Gorth et al. evaluates in vitro the therapeutic potential of PLGA microspheres loaded with an interleukin-1 receptor antagonist (IL-1ra) for sustained attenuation of IL-1$\beta$ mediated degradation of the nucleus pulposus (NP), a key step in the inflammatory processes derived from intervertebral disc degeneration that cause back pain.

The document entitled Cannabinoids delivery systems based on supramolecular inclusion complexes and polymeric nanocapsules for treatment of neuropathic pain (Astruc-Diaz, 2012) describes the design of four delivery systems for the cannabinoid drug CB2 agonist MDA7. Specifically: (i) micellar solutions; (ii) self-emulsifying systems; (iii) choline liposomes; and (iv) cyclodextrin inclusion complexes for parenteral administration.

The authors have conducted in vivo pK/pD studies and have compared the effectiveness of the designed systems in a rat model of neuropathic pain. The delivery systems based on cyclodextrins were the most promising.

In addition, the document describes the design of cationic nanoparticles obtained by nanoprecipitation using Eudragit® RS100/RL100 polymers. In the latter case, they use another CB2 agonist, beta-caryophyllene [(E)-BCP], as a model drug.

In no case have the cannabinoid drugs used, MDA7 and (E)-BCP, been conveyed in PLGA nanoparticles.

A document in the Journal of Research in Medical Sciences (2012) by Abrishamkar et al. shows the results of a randomized clinical trial involving 38 patients, in which bupivacaine conjugated to N--Methyl-2pyrolidone-ethyl Heptanoate (PLGA) is administered locally to relieve radicular and lower back pain following lumbar discectomy. The trial showed the capacity of this system to relieve pain for 4 weeks following surgical intervention. The drug was not conveyed in PLGA nanoparticles in any way.

A document in J Biomater Appl. (2013) by Chen et al. describes obtaining, by means of nanoprecipitation, of loperamide nanoparticles and the poly(lactic-co-glycolic)-poly(ethylene glycol)-poly(lactic-co-glycolic) copolymer (PLGA-PEG-PLGA) coated with poloxamer 188 or polysorbate 80 surfactant with the aim of passing through the blood-brain barrier and obtaining an antinociceptive effect. Loperamide in and of itself does not pass through the blood-brain barrier. This study demonstrated the ability of these nanoparticles to pass through it, obtaining a maximum analgesic effect of 21-35% 150 minutes after intravenous administration. The document in no case describes the administration of pegylated PLGA particles for oral administration.

A document in J Nanopart Res (2011) by Garcia et al. describes an injectable formulation based on PLGA nanoparticles containing the bupivacaine anesthetic with the aim of obtaining an extended analgesic effect in the treatment of nociceptive and neuropathic pain. Nanoparticles with a diameter of 453 nm were obtained by triple emulsion and were administered locally through infiltration to rats with sciatic nerve constriction as a model of mononeuropathy.

The results of the study showed that the system administered locally and based on bupivacaine nanoparticles was much more effective since it maintained the analgesic and antialodynic effect for a longer period of time than when a solution of the drug was administered. In no case does the document describe the administration of pegylated PLGA particles for oral administration.

A document in J Nanosci Nanotechnol. (2011) by Kim et al., describes the use of PLGA nanoparticles and microparticles containing capsaicin. The nanoparticles were obtained by nanoprecipitation with sizes ranging from 152-163 nm in diameter. In order to further control the delivery and increase the encapsulation efficiency of the drug, modifications were made to the production method by using other biodegradable polymers such as polycaprolactone (PCL). As a result, formulations of capsaicin capable of controlling their delivery from 1 week to 1 month were obtained. In no case does the document describe the administration of pegylated PLGA particles for oral administration.

The document entitled Nanoparticles with biodegradable and biocompatible polymer PLGA, loaded with the drug for human use pentoxifylline by Constandil Córdova (WO2014075203) describes biodegradable and biocompatible nanoparticles for administering the drug pentoxifylline for use in the treatment and prevention of chronic pain via the administration of a single dose. The document relates to both the synthesis method and the use thereof and claims the pharmaceutical formulation with PLGA particles with a variable ratio of lactic acid/glycolic acid ranging from 10% to 90% and vice versa, with a homogeneous size between 150 and 410 nm, a negative surface charge with a value between -24 and -13 mV, an encapsulation efficiency of between 40 and 60%, the ratio of encapsulated pentoxifylline is 1-3 $\mu$g in 10$\mu$L of particle solution. Its may be administered intrathecally, intravenously or intramuscularly. The document does not disclose the particular use of PLGA and/or PEG with CB13 nor does it disclose a route of oral administration.

The document entitled Polymeric particles-based temozolomide dosage form by Vasilenko et al.,(WO2014091078A1) discloses a controlled delivery system of the anti-cancer drug temozolomide (TMZ) based on PLGA nanoparticles. The formulation includes an active material, polyvinyl alcohol or serum albumin on the surface and a cryoprotectant (D-mannitol or glucose). The document describes obtaining the nanoparticles through emulsion-evaporation of the solvent with an average diameter of 200-500 nm. The nanoparticles obtained for oral administration in the form of capsules or tablets or daily parenteral administration in the form of a sterile suspension reduce the toxicity of the free drug.

The document entitled Microparticles and nanoparticles having negative surface charges by Bin (WO2014089160) describes the production of PLGA nanoparticles and microparticles with negative surface charges. The particles are made from the emulsion-evaporation method of the solvent. The particles have a zeta potential of from -5 to -200 mV, preferably, -35 to -85 mV and sizes in the range of 10-1000 nm. The particles can be PEGylated through three processes: (i) absorption of PEG on the surface of the particles; (ii) mixing PEG and PLGA during synthesis; or (iii) covalently bonding to PLGA. The purpose of the particles is the treatment of inflammatory illnesses with an immune response through their oral or parenteral administration.

PLGA has been widely used for encapsulating drugs, however, there are no known disclosures of an oral formulation for the treatment of neuropathic pain caused by peripheral nerve compression corresponding to a PLGA-PEG nanoparticle containing cannabinoid drugs for the oral administration thereof.

**Bibliographical references**

Haanpää M, Attal N, Backonja M, Baron R, et al., NeuPSIG guidelines on neuropathic pain assessment. Pain. 2011;152(1):14-27. doi: 10.1016/j.pain.2010.07.031.

Abrishamkar S, Torkashvand M, Borujeni AM, Borujeni AM, Rezvani M, Tabesh H, et al. Efficacy of N-Methyl-2Pyrolidone-Ethyl heptanoate (PLGA)-bupivacaine in situ forming system on radicular and low back pain relief following lumbar discectomy: A randomized clinical trial. Journal of Research in Medical Sciences 2012;17:S165-S170.

Astruc-Diaz F., Cannabinoids delivery systems based on supramolecular inclusion complexes and polymeric nanocapsules for treatment of neuropathic pain. Doctoral Thesis, Lyon (France), 2012.

Bin W. Microparticles and nanoparticles having negative surface charges. WO2014089160.

Boisseau P, Kiparissides C, Pavesio A, Saxl O, Nanotechnology for Health. Vision Paper and Basis for a Strategic Research Agenda for Nanomedicine. European Technology Platform on Nanomedicine. European Commission (2005).

Bossart J, Sedo K, Kararli T. Delivery Report. Drug Delivery Products & Technologies, a Decade in Review: Approved Products 2000 to 2009 Chen Y, Hsieh W, Lee W, Zeng D. Effects of surface modification of PLGA-PEG-PLGA nanoparticles on loperamide delivery efficiency across the blood-brain barrier. J Biomater Appl 2013;27(7):909-922.

Constandil Córdova LE, Ibarra Durán PS, Vilos Ortiz CA, Velásquez Cumplido L, Pelissier Serrano T, Laurido Fuenzalida CA, Hernández Kunstmann A. Nanoparticles with biodegradable and biocompatible polymer PLGA, loaded with the drug for human use pentoxifylline WO2014075203.

Dziadulewicz EK, Bevan SJ, Brain CT, Coote PR, Culshaw AJ, et al.,Naphthalen-1-yl-(4-pentyloxynaphthalen-1-yl)methanone: A potent, orally bioavailable human CB1/CB2 dual agonist with antihyperalgesic properties and restricted central nervous system penetration. J. Med. Chem. 50, 3851 (2007).

Garcia X, Escribano E, Domenech J, Queralt J, Freixes J. In vitro characterization and in vivo analgesic and anti-allodynic activity of PLGA-bupivacaine nanoparticles. Journal of Nanoparticle Research 2011;13(5):2213-2223.

Gardin A., Kucher K, Kiese B, and Appel-Dingemanse S. Cannabinoid receptor agonist 13, a novel cannabinoid agonist: First in human pharmacokinetics and safety. Drug Metab. Dispos. 37, 827 (2009)

Gorth DJ, Mauck RL, Chiaro JA, Mohanraj B, Hebela NM, Dodge GR, Elliott DM, Smith LJ. IL-1ra delivered from poly(lactic-co-glycolic acid) microspheres attenuates IL-1β-mediated degradation of nucleus pulposus in vitro. Arthritis Res Ther. 2012 3;14(4):R179. doi: 10.1186/ar3932.

Hudson and Margaritis A. Biopolymer nanoparticle production for controlled release of biopharmaceuticals. June 2014, Vol. 34, No. 2, Pages 161-179

Jain K, Goyala A, Mishra N, Vaidya B, Mangal S, Vyas SP. PEG-PLA-PEG block copolymeric nanoparticles for oral immunization against hepatitis B. International Journal of Pharmaceutics 387 (2010) 253-262 Kim S, Kim JC, Sul D, Hwang SW, Lee SH, Kim YH, et al. Nanoparticle Formulation for Controlled Release of Capsaicin. Journal of Nanoscience and Nanotechnology 2011;11(5):4586-4591.

Lai P, Daear W, Löbenberg R, Prenner EJ. Overview of the preparation of organic polymeric nanoparticles for drug delivery based on gelatine, chitosan, poly(d,l-lactide-co-glycolic acid) and polyalkylcyanoacrylate. Colloids and Surfaces B: Biointerfaces 118(1), 2014, 154-163

Lalani J, Rathi M, Lalan M, Misra A. Protein functionalized tramadol-loaded PLGA nanoparticles: preparation, optimization, stability and pharmacodynamic studies. Drug Dev Ind Pharm 2013;39(6):854-864.

Lam PL, Gambari R. Advanced progress of microencapsulation technologies: In vivo and in vitro models for studying oral and transdermal drug deliveries Journal of Controlled Release 178 (2014) 25-45

Martin-Banderas L, Josefa Alvarez-Fuentes, Matilde Durán-Lobato, Jose Prados, Consolación Melguizo, Mercedes Fernandez-Arevalo, Ma Angeles Holgado. Cannabinoid derivate-loaded PLGA nanocarriers for oral administration: formulation, characterization, and cytotoxicity studies. International Journal of Nanomedicine 2012:7 5793-5806

Milligan ED, Penzkover KR, Soderquist RG, Mahoney MJ. Spinal Interleukin-10 Therapy to Treat Peripheral Neuropathic Pain. Neuromodulation 2012;15(6):520-526.

Nocerino E, Amato M, Izzou AA (2000), "Cannabis and cannabinoid receptor", Fitoterapia, 71:6-12.

Ratajczak-Enselme M, Estebe J, Dollo G, Chevanne F, Bec D, Malinovsky J, et al. Epidural, intrathecal and plasma pharmacokinetic study of epidural ropivacaine in PLGA-microspheres in sheep model. European Journal of Pharmaceutics and Biopharmaceutics 2009;72(1):54-61.

Shen H, Hu X, Szymusiak M, Wang ZJ, Liu Y. Orally Administered Nanocurcumin to Attenuate Morphine Tolerance: Comparison between Negatively Charged PLGA and Partially and Fully PEGylated Nanoparticles. Molecular Pharmaceutics 2013;10(12):4546-4551. Vasilenko E, ; Vorontsov E, ; Severin E, ; Gulenko V, Mitrokhin M, ; Iurchenko, M. Polymeric particles-based temozolomide dosage form WO2014091078.

Vila A, Sanchez A, Tobío M, Calvo P, Alonso MJ. Design of biodegradable particles for protein delivery. Journal of Controlled Release 78 (2002) 15-24

Wacker M. Nanocarriers for intravenous injection-The long hard road to the market. International Journal of Pharmaceutics Volume 457, Issue 1, 2013, Pages 50-62

Zanella JM, Burright EN, Hildebrand K, Hobot C, Cox M, Christoferson L, et al. Effect of etanercept, a tumor necrosis factor-alpha inhibitor, on neuropathic pain in the rat chronic constriction injury model. Spine 2008;33(3):227-234. http://www.marketsandmarkets.com/Market-Reports/drug-delivery-technologies-market-1085.html, September, 2014.

Owens DE, Peppas NA. Opsonization, biodistribution, and pharmacokinetics of polymeric nanoparticles. International Journal of Pharmaceutics: 2006; 307(1), 93-102.

**Description of the Figures**

Figure 1: (A) Size and size distribution of empty and CB13-loaded PLGA-PEG particles (B) Zeta potential of CB13-loaded and empty particles

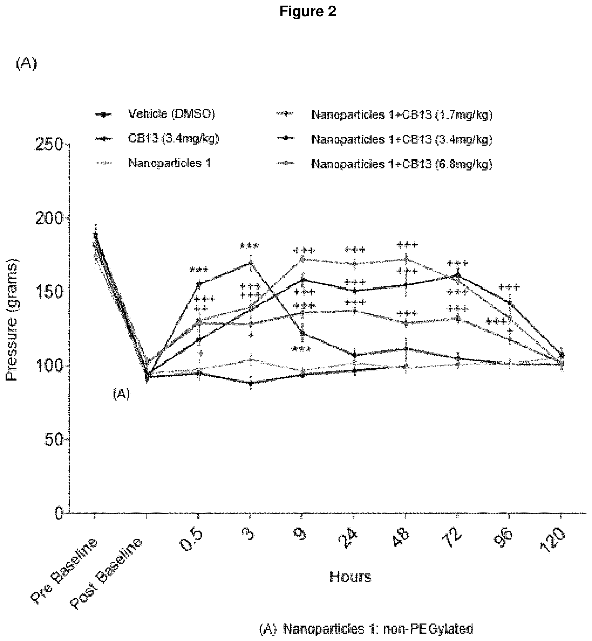

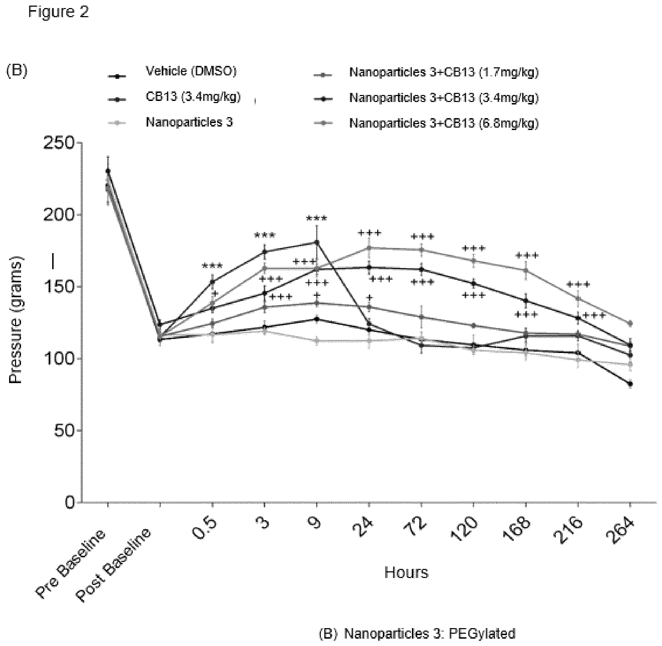

Figure 2. Antinociceptive effect of different tested formulations after a single oral administration of empty PLGA nanoparticles and CB13-loaded nanoparticles at three different doses. (A) non-pegylated PLGA nanoparticles. (B) PLGA-PEG nanoparticles (covalently bonded) (carrier: DMSO)

**Description of the Invention**

The present invention discloses a new pharmaceutical formulation the final composition of which combines the cannabinoid drug CB13, the design of a nanoparticle based on the lactic and glycolic acid (poly(lactic-co-glycolic acid)) copolymer (PLGA), an active material on the surface of the particle and an cryoprotectant and the use thereof for the effective treatment of neuropathic pain caused by peripheral nerve compression by administering a single oral dose.

In a preferred embodiment, the nanoparticles are synthesized by the nanoprecipitation method, which comprises the following steps:

    a) Dissolving the PEG-PLGA polymer in a solvent
    b) Dissolving a lipophilic surfactant in the previous solution
    c) Dissolving the drug in the previous solution
    d) Dissolving a hydrophilic surfactant in purified water
    e) Adding the drug-polymer co-solution (a+b+c) to the surfactant solution (d) drop by drop
    f) Evaporating the solvent where the drug and the polymer were dissolved
    g) Washing the nanoparticles with purified water
    h) Collecting the nanoparticles
    i) Adding a cryoprotectant
    j) Conserving the nanoparticles by freezing

The CB13-loaded nanoparticles of the present invention are useful for relieving neuropathic pain caused by peripheral nerve compression by orally administering a single dose.

The pharmaceutical formulation having slow, controlled and sustained delivery of the CB13-loaded PEG-PLGA nanoparticles of the present invention comprises the suspension of CB13-loaded PEG-PLGA nanoparticles obtained from the method previously described and any suitable additive, agent, excipient or adjuvant.

The present invention discloses a new pharmaceutical formulation comprising two biodegradable polymers: the poly(lactic-co-glycolic acid) copolymer (PLGA) and the polyethylene glycol (PEG); the cannabinoid drug CB13 and the use for the effective treatment of neuropathic pain caused by peripheral nerve compression by orally administering a single dose.

Polyester nanoparticles such as PLGA are suitable as controlled and sustained drug delivery systems given their delivery profile, their high biocompatibility and the fact that their degradation products are absorbed. The presence of PEG chains in the nanoparticles, in turn, confers greater stability in acidic environments, such as the stomach, prevents the absorption of proteins and prevents opsonization by the macrophages, increasing the time of systemic circulation.

The present invention uses the drug 1-naphthalenyl[4-(pentyloxy)-1-naphthalenyl]methanone (CB13, Novartis), a new CB1/CB2 receptor dual agonist, exhibiting powerful antihyperalgesic activity in animal models and limited penetration into the brain. However, the drug belongs to Class II of the Biopharmaceutics Classification System (BCS), having low aqueous solubility. Therefore, in order to be orally administrated in the studies that have demonstrated its drug activity, micellar solutions had to be used, using emulsifying and solubilizing agents such as Chremophor® (non-ionic agent based on castor oil and ethylene oxide) and/or Labrafil® M2125CS. These micellar systems administered orally provided analgesia for a maximum of 8 hours based on the dose administered.

The use of CB13-loaded PLGA nanoparticles solves this problem since it allows the slow, controlled and sustained delivery of the drug as the particle degrades and/or erodes, meaning that the administration of a single oral dose has an effect that relieves chronic pain for days.

The CB13-loaded PLGA nanoparticles of the present invention are synthesized by any method known in the state of the art, for example, but not limited to, simple emulsion or double evaporation of the solvent; method for separating the polymer or nanoprecipitation; or, traditional or modified spray-drying method. In a preferred embodiment, the particles are synthesized by the nanoprecipitation method comprising the following steps:

    a) Dissolving the PEG-PLGA polymer in a solvent;
    b) Dissolving a lipophilic surfactant in the previous solution;
    c) Dissolving the drug in the previous solution;
    d) Dissolving a surfactant in purified water;
    e) Adding the drug, polymer and drug solution (a+b+c) to the surfactant solution (d) drop by drop and under continuous stirring;
    f) Evaporating at room temperature the solvent wherein the drug and the polymer were dissolved;
    g) Washing the nanoparticles with purified water;
    h) Collecting the nanoparticles;
    i) Adding a cryoprotectant;
    j) Conserving the nanoparticles by freezing.

The PEG-PLGA polymer used in step a) and therefore the nanoparticles of the present invention have a ratio of lactic acid to glycolic acid ranging from 10% lactic acid and 90% glycolic acid to 90% lactic acid and 10% glycolic acid, any proportion therebetween being possible. In a preferred embodiment, the proportion is 50% lactic acid and 50% glycolic acid.

The molecular weight of the PEG included can vary from 2,000 to 20,000 Da. In a preferred preparation it is 2,000 Da. The PEG is covalently bonded to PLGA.

The solvent used to dissolve the polymer in step a) is any that allows the polymer to be dissolved, for example, but not limited to, acetone or acetonitrile.

The polymer:drug ratio used in the synthesis method of the present invention ranges from 99:1, 95:5, 90:10, 85:15, being any combination that is around this interval. In a preferred use, the polymer:CB13 ratio is 90:10 and 85:15.

The surfactant used in step b) may be any that is lipophilic and allows isolated and non-added particles to form, for example, but not limited to, Span@ 60 and the derivatives thereof.

In a preferred embodiment, when Span@ 60 is used in step b), the Span@ 60 solution in the solvent of step a) comprises 0.1 - 5.0% w/v. In an even more preferred embodiment, the Span@ 60 solution in the solvent of step a) comprises 0.5% w/v of Span@ 60.

Step e) of adding (a+b+c) to d) is carried out with any method known in the state of the art, being able to manually use a suitable syringe and needles or using a system that controls the rate of adding one phase to another. In a preferred embodiment, a syringe pump is used at a rate of, but not limited to, 5 mL/h, with a syringe and a yellow needle.

Stirring in step e) is carried out, but not limited to, magnetic stirring, meaning it can be mechanical and at a rate of, but not limited to, 300-1000 rpm. In a preferred embodiment, stirring will be magnetic, at room temperature and at a rate of 600 rpm.

The surfactant used in step d) may be any one that allows the surface tension of the water to be reduced and allows the formation of isolated and non-aggregated particles, for example, but not limited to, Pluronic® F68 and derivatives thereof; polyethylene glycol and the derivatives thereof, polyvinyl alcohol, cationic or anionic emulsifiers for pharmaceutical use or any combination thereof.

In a preferred embodiment, when Pluronic® 68 is used in step d), the Pluronic® 68 solution in purified water comprises 0.1%-5.0% w/v. In an even more preferred embodiment, the Pluronic® 68 solution in purified water in step d) comprises 0.5% w/v of Pluronic® F68.

Evaporating the solvent of step a) is carried out through any method known in the state of the art, for example, but not limited to: stirring the particle suspension; gas streams such as nitrogen or oxygen, heat, creating a vacuum, freeze-drying or any combination thereof. In a particular embodiment, evaporating the solvent is carried out at room temperature for 4-5 hours by orbital stirring.

Collecting particles in suspension of step h) is carried out by any known method, for example, but not limited to: filtration; filtration and centrifugation; differential centrifugation; gradient centrifugation; or any combination thereof. In a particular embodiment, the particles are collected by centrifugation at 4-12°C, 9,000-11,000 rpm for 45-60 minutes. In an even more particular embodiment, the particles are collected by centrifugation at 4°C, 11,000 rpm for the time required to collect a concentrated suspension of nanoparticles.

The method for collecting CB13-loaded PEG-PLGA nanoparticles of the present invention allows recovering between 60-80% of the weight of the particles. In a preferred embodiment, the efficiency of the collection process is 70% of the weight of the particles.

The CB13-loaded PEG-PLGA nanoparticles of the present invention have a homogeneous size with an average diameter between 145-450 nm. In a preferred embodiment, the CB13-loaded PEG-PLGA nanoparticles of the present invention have an average diameter of 180 nm.

The CB13-loaded PEG-PLGA nanoparticles of the present invention are spherical and have a negative electric surface charge with a value between -35 mV and -20 mV. In a preferred embodiment, the CB13-loaded PEG-PLGA nanoparticles of the present invention have an electric surface charge of -26 mV.

The method for synthesizing the CB13-loaded PEG-PLGA nanoparticles of the present invention allows encapsulation efficiency between 70% and 90% of the added drug. In a preferred embodiment, the encapsulation efficiency is 80% of the drug.

The ratio of encapsulated CB13 in the PEG-PLGA particles of the present invention is between 9-15% w/w. In a preferred embodiment, the ratio of encapsulated CB13 is 13% w/w drug/polymer.

*Advantages of the present invention*

The present invention solves the problem of the need for permanent administration of drugs for the treatment of neuropathic pain caused by peripheral nerve compression.

The present invention uses a single oral dose of a pharmaceutical formulation comprising at least one aqueous solution of biodegradable and biocompatible PLGA polymeric nanoparticles loaded with the drug CB13 to treat neuropathic pain caused by peripheral nerve compression. It can further comprise any suitable additive, adjuvant or pharmaceutical agent.

**Embodiment of the Invention**

*Example 1. Synthesis and characterization of CB13-loaded polymeric nanoparticles.*

The nanoparticles were prepared by the modified method of nanoprecipitation described by Fessi et al. (1989).

The type of PLGA-PEG (Sigma) used is made up of 50% glycolic acid and 50% lactic acid.

The polymer was dissolved into 2 mL of acetone (1.5% w/v) along with the Span®60 surfactant (0.5% w/v). Once dissolved, 7.5 mg of the drug was added. Next, this solution was added drop by drop at a constant rate of 5 mL/h by a syringe pump (Harvard Apparatus) onto 15 mL of Pluronic® F68 solution (0.5% w/v) under magnetic stirring (600 rpm, IKA) at room temperature.

The acetone was evaporated by gentle stirring for 4 hours at room temperature. After this process, the nanoparticles were washed with Milli-Q water and collected by ultracentrifugation (4°C, 60 min, 10,000 rpm, Eppendorf). Finally, the collected pellet was re-suspended in 1 mL of a trehalose solution (VWR) at 5% w/v and stored at -20°C.

To control the production process of the nanoparticles, empty, drug-free particles were prepared using the same methods as those described above.

The size and zeta potential of the nanoparticles were measured through dynamic light scattering (DLS) (Zetasizer Nano Z, Malvern, United Kingdom). For this purpose, a 100 uL aliquot of the nanoparticle suspension was taken and brought to a volume of 1 mL of Milli-Q water for analysis by DLS.

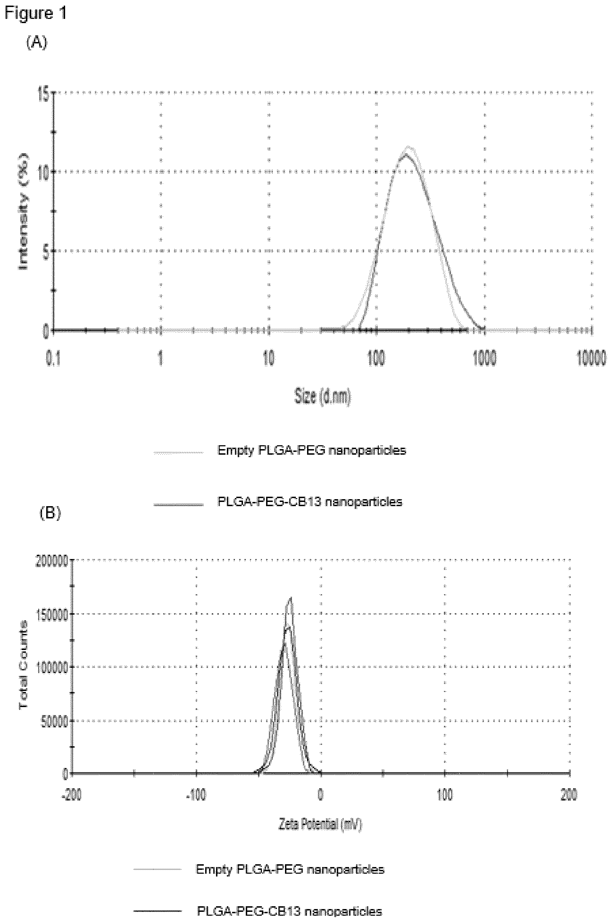

The PLGA-PEG nanoparticles showed a particle size of 200.54 $\pm$ 28.80 nm (n=6), and showed no significant differences with respect to the empty particles, as seen in Figure 1A.

Figure 1B shows the surface charge of the loaded and empty particles. As can be observed, there are essentially no differences in the values. The particles showed a negative potential of -26.95 $\pm$ 1.94 mV (n=6).

*Example 2. Encapsulation Efficiency and Drug Loading Capacity*

The encapsulation efficiency (EE%) and drug loading capacity (DLC) of the particles were determined by quantitatively comparing the initial amount of the drug and the amount extracted after breaking a certain number of nanoparticles (5 mg) after dissolution thereof in 1 mL of acetone.

The amount of drug was quantified by reverse-phase high-performance liquid chromatography (RP-HPLC) in Hitachi LaChrom® Series D-7000 (Hitachi Ltd, Tokyo, Japan) system equipment, fitted with an automatic injector (L-7200), a D-7000 interphase, a quaternary pump (model L-7100), and a UV-VIS DAD detector (model L-7455). A Spherisorb ODS2 column (Waters Corp) (10 $\mu$m, 4.6 mm $\times$ 250 mm) was used at 40.0°C $\pm$ 0.1°C (Elite LaChrom L-2350 oven). The data was collected and analyzed using HSM D-7000 LaChrom® software (Hitachi, Ltd).

A mobile phase made up of two solvents was used to chromatographically separate CB13.

- Solvent A: acetonitrile: water: acetic acid (75:23.7:1.3 v/v)
- Solvent B: acetonitrile

The solvents were maintained at a ratio of 70%: 30% (A: B) at a constant flow of 1.00 mL/min.

The wavelength was set at 230 nm and the injection volume at 10 $\mu$L.

EE% was determined using the following formula:

$$EE\% = (\text{current amount of CB13 in the nanoparticles} / \text{theoretical amount of CB13 in the nanoparticles}) \times 100$$

CC was determined using the following formula:

$$CC\% = (\text{current mass of CB13 in the nanoparticles} / \text{mass of nanoparticles}) \times 100$$

The encapsulation efficiency results showed that 80% of the drug added during the process of preparing the nanoparticles was included therein. The load capacity was calculated from these results.

Since 30% of the particle mass is lost during the process of washing and collecting the particles and 80% of the drug initially used is encapsulated, the amount of particles to be used in the in vivo trials, in which three different doses were fixed, is then calculated.

*Example 3. Determining analgesic properties and in vivo comparison of the nanoparticles. Animals*

Harlan Sprague-Dawley male albino rats (230-350 g), provided by the University of Cádiz Animal Experimentation Unit registered under the number ES110120000210, were used. The animals were kept under standard housing conditions (21°C, 12h light-dark cycle, "ad libitum" water and food). All experiments were carried out in accordance with the ethical guidelines for working with experimental animals proposed by the International Association for the Study of Pain (IASP) and by the Federation of Laboratory Animal Science Associations (FELASA).

*Neuropathic pain models*

Chronic constriction injury, (CCI) was used as a model of mononeuropathy since it is a model that correlates well with the symptoms presented in the patients (spontaneous pain, mechanical and thermal allodynia and hyperalgesia.

*Assessment of nociceptive behavior*

Nociceptive behavior was evaluated in the ipsi- and contralateral paw to the ligation for sciatic nerve injection. The presence of mechanical hyperalgesia was assessed by using the paw-pinch test. Thus, gradual, increasing pressure (starting with 30 g of pressure) was exerted on the rear part of the paw using a motorized device (Analgesia Meter 57215, UGO BASILE). The cut-off pressure was set at 250 g to prevent possible damage to the paw. Two measurements were taken on each paw, 5 minutes apart, and the average value was used for statistical purposes. Mechanical hyperalgesia appeared as the pressure on the paw decreased.

As expected, the control groups (Vehicle-DMSO and Nanoparticle) showed hyperalgesic behavior that remained constant throughout the experiment (Fig. 1A and 1B)

The anti-hyperalgesic effect of CB13 was evident within 30 minutes of its administration and presented its maximum effect between 30 minutes and 3 hours. 9 hours after its administration, CB13 continued to be effective but to a lesser extent, and after 24 hours, its effectiveness had disappeared. With regard to the combination of non-PEGylated nanoparticles (Fig. 1A) with three increasing doses of CB13 (1.7-6.8 mg/kg), we can observe a clearly dose-dependent analgesic effect. The effect of the non-PEGylated nanoparticles and the different doses of CB13 became evident within 30 minutes of its administration; however, it was smaller than the CB13 alone. On the other hand, the analgesic effect lasted up to 96 hours (4 days).

The analgesic effect of the CB13 group was very similar to the previous experimental group. With regard to the combination of PEGylated Nanoparticles 3 (Fig. 1B) with the different doses of CB13, we can observe that the analgesic effect appeared 30 minutes after administration in the group of Nanoparticles 3+CB13 (6.8 mg/kg) and within 3 hours in the group with Nanoparticles 3+CB13 (3.4 mg/kg). This analgesic effect of both groups lasted up to 9 days after administration. The group of Nanoparticles 3+CB13 (1.7 mg/kg) had a statistically significant analgesic effect within 9 and 24 hours after administration.

**Claims**

1. A method for obtaining nanoparticles from the poly(lactic-co-glycolic acid) copolymer (PLGA) and the polyethylene glycol (PEG) with the encapsulated cannabinoid drug CB13, **characterized in that** it comprises the following steps: a) Dissolving the PEG-PLGA polymer in a solvent; b) Dissolving a lipophilic surfactant in the previous solution; c) Dissolving the drug in the previous solution; d) Dissolving a surfactant in purified water; e) Adding the drug-polymer co-solution (a+b+c) to the surfactant solution (d) drop by drop and under continuous magnetic stirring; f) Evaporating at room temperature the solvent wherein the drug and the polymer were dissolved; g) Washing the nanoparticles with purified water; h) Collecting the nanoparticles; i) Adding a cryoprotectant; j) Conserving the nanoparticles by freezing.

2. The method according to claim 1, **characterized in that** the PEG-PLGA polymer has a ratio of lactic acid to glycolic acid ranging from 10% lactic acid and 90% glycolic acid to 90% lactic acid and 10% glycolic acid, any proportion therebetween being possible.

3. The method according to the preceding claims, **characterized in that** the molecular weight of the PEG varies from 2,000 to 20,000 Da.

4. The method according to the preceding claims, **characterized in that** the ratio of the polymer to the drug varies in a range from 99:1 to 85:15.

5. The method according to the preceding claims, **characterized in that** the surfactant in step (d) is a lipophilic surfactant.

6. The method according to claim 5, **characterized in that** the lipophilic surfactant is selected from among polyethylene glycol and the derivatives thereof, polyvinyl alcohol and the derivatives thereof, cationic or anionic emulsifiers for pharmaceutical use or any combination thereof.

7. The method according to the preceding claims, **characterized in that** the magnetic stirring takes place at a rate of 600 rpm.

8. The method according to the preceding claims, **characterized in that** the evaporation is carried out by stirring the particle suspension, gas streams such as nitrogen or oxygen, heat, creating a vacuum, freeze-drying or any combination thereof.

9. The method according to the preceding claims, **characterized in that** the evaporation at room temperature under mechanical stirring at 600 rpm takes place for 4 hours.

10. The method according to the preceding claims, **characterized in that** collecting particles in suspension is carried out by: filtration; filtration and centrifugation; differential centrifugation; gradient centrifugation; or any combination thereof.

11. The method according to the preceding claims, **characterized in that** the particles are collected by centrifugation at 4°C and at 10,000 rpm for the time required to obtain a nanoparticle concentrate.

12. A pharmaceutical composition, **characterized in that** it comprises poly(lactic-co-glycolic acid) (PLGA) nanoparticles with encapsulated CB13.

13. The pharmaceutical composition according to claim 12, **characterized in that** the CB13-loaded nanoparticle has a ratio of lactic acid to glycolic acid ranging from 10% lactic acid and 90% glycolic acid to 90% lactic acid and 10% glycolic acid, any proportion therebetween being possible..

14. The pharmaceutical composition according to claims 12 and 13, **characterized in that** the CB13-loaded nanoparticle has a ratio of 50% lactic acid and 50% glycolic acid and a homogeneous size with an average diameter between 145-450 nm.

15. The pharmaceutical composition according to claims 12 to 14, **characterized in that** the CB13-loaded PEG-PLGA nanoparticles of the present invention are spherical.

16. The pharmaceutical composition according to claims 12 to 15, **characterized in that** the CB13-loaded PEG-PLGA nanoparticles have a negative electric surface charge with a value between -35 mV and -20 mV.

17. The pharmaceutical composition according to the claim 12 to 16, **characterized in that** the ratio of encapsulated CB13 in the PEG-PLGA nanoparticles is 9-15% w/w.

18. The pharmaceutical composition according to claims 12 to 17, **characterized in that** the single slow, controlled and sustained delivery dose of the CB13-loaded PEG-PLGA nanoparticles is in the range of 0.1-10.0 mg of CB13 per kilogram of body weight.

Figure 1

(A)

Empty PLGA-PEG nanoparticles

PLGA-PEG-CB13 nanoparticles

(B)

Empty PLGA-PEG nanoparticles

PLGA-PEG-CB13 nanoparticles

**Figure 2**

(A)

(A) Nanoparticles 1: non-PEGylated

Figure 2

(B) Nanoparticles 3: PEGylated

## EP 3 257 503 A1

## INTERNATIONAL SEARCH REPORT

| International application No. |
|---|
| PCT/ES2016/000016 |

### A. CLASSIFICATION OF SUBJECT MATTER

*A61K9/52* (2006.01)
*B82Y5/00* (2011.01)
According to International Patent Classification (IPC) or to both national classification and IPC

### B. FIELDS SEARCHED

Minimum documentation searched (classification system followed by classification symbols)
A61K, B82Y

Documentation searched other than minimum documentation to the extent that such documents are included in the fields searched

Electronic data base consulted during the international search (name of data base and, where practicable, search terms used)

EPODOC, INVENES, WPIAP,TCM, TXPEA-C,TXPEE,TXPEF,TXPEH,TXPEI,TXPEP,TXPEPEA,TXTPES,TXPUS,TXPWOEA BIOSIS, MEDLINE, XPESP, XPESP2, NPL, EMBASE

### C. DOCUMENTS CONSIDERED TO BE RELEVANT

| Category* | Citation of document, with indication, where appropriate, of the relevant passages | Relevant to claim No. |
|---|---|---|
| X | DURÁN-LOBATO, M., MARTÍN-BANDERAS, L., GONÇALVES, L. et al. Comparative study of chitosan-and PEG-coated lipid and PLGA nanoparticles as oral delivery systems for cannabinoids. Journal of nanoparticle research (30.01.2015). Vol 17, N° 2, pages 1-17, ISSN 1388-0764 | 1-18 |
| A | ANAND, P., NAIR, H. B., SUNG, B. et al. Design of curcumin-loaded PLGA nanoparticles formulation with enhanced cellular uptake, and increased bioactivity in vitro and superior bioavailability in vivo. Biochemical Pharmacology (01.02.2010), Vol 79, pages 330-338 | 1-18 |

☒ Further documents are listed in the continuation of Box C.   ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 27/04/2016 | **(29/04/2016)** |

| Name and mailing address of the ISA/ | Authorized officer |
|---|---|
| OFICINA ESPAÑOLA DE PATENTES Y MARCAS Paseo de la Castellana, 75 - 28071 Madrid (España) Facsimile No.: 91 349 53 04 | A. Barrios de la Fuente Telephone No. 91 3496868 |

Form PCT/ISA/210 (second sheet) (January 2015)

16

**INTERNATIONAL SEARCH REPORT**

| International application No. |
| --- |
| PCT/ES2016/000016 |

| C (continuation). | DOCUMENTS CONSIDERED TO BE RELEVANT | |
| --- | --- | --- |
| Category * | Citation of documents, with indication, where appropriate, of the relevant passages | Relevant to claim No. |
| A | TANG, LI., AZZI, J., KWON, M. et al. Immunosuppressive Activity of Size-Controlled PEG-PLGA Nanoparticles Containing Encapsulated Cyclosporine A. Journal of transplantation (2012). ISSN 2090-0015 (Electronic), pages 1-9 | 1-18 |
| A | KARVE, S., WERNER, M.E., CUMMINGS, N.D et al .Formulation Of Diblock Polymeric Nanoparticles through Nanoprecipitation Technique. Journal of Visualized experiments (20/09/2011). Retrieved of internet. URL: < http://www.jove.com/video/3398/formulation-diblock-polymeric-nanoparticles-through-nanoprecipitation> | 1-18 |
| A | HANS, M. L, LOWMAN, A. M. Biodegradable nanoparticles for drug delivery and targeting. Current Opinion in Solid State and Materials Science (08.2002), Vol 6, N° 4. Pages 319-327, doi:10.1016/S1359-0286(02)00117-1 | 1-18 |

Form PCT/ISA/210 (continuation of second sheet) (January 2015)

## REFERENCES CITED IN THE DESCRIPTION

### Patent documents cited in the description

- WO 2014075203 A
- WO 2014091078 A1
- WO 2014089160 A
- WO 2014091078 A
- ES 110120000210

### Non-patent literature cited in the description

- **MARTIN-BANDERAS.** *International Journal of Nanomedicine,* 2012, vol. 7, 5793-5806
- **SHEN.** *Mol. Pharm.,* 2013, vol. 10, 4546-4551
- **LALANI.** *Drug Dev.Ind.Pharm.,* 2013, vol. 39 (6), 854-864
- **MILLIGAN.** *Neuromodulation,* 2012, vol. 15 (6), 520-6
- **RATAJCZAK-ENSELME M.** *Eur J Pharm Biopharm,* 2009, vol. 72 (1), 54-61
- **ZANELLA.** *Spine,* 2008, vol. 33 (3), 227-234
- **GORTH.** *Arthritis Research & Therapy,* 2012, vol. 14 (4), R179
- **ABRISHAMKAR.** *Journal of Research in Medical Sciences,* 2012
- **CHEN.** *J Biomater Appl.,* 2013
- **GARCIA.** *J Nanopart Res,* 2011
- **KIM.** *J Nanosci Nanotechnol.,* 2011
- **HAANPÄÄ M ; ATTAL N ; BACKONJA M ; BARON R et al.** NeuPSIG guidelines on neuropathic pain assessment. *Pain.,* 2011, vol. 152 (1), 14-27
- **ABRISHAMKAR S ; TORKASHVAND M ; BORUJENI AM ; BORUJENI AM ; REZVANI M ; TABESH H et al.** Efficacy of N-Methyl-2Pyrolidone-Ethyl heptanoate (PLGA)-bupivacaine in situ forming system on radicular and low back pain relief following lumbar discectomy: A randomized clinical trial. *Journal of Research in Medical Sciences,* 2012, vol. 17, S165-S170
- **ASTRUC-DIAZ F.** Cannabinoids delivery systems based on supramolecular inclusion complexes and polymeric nanocapsules for treatment of neuropathic pain. *Doctoral Thesis,* 2012
- **BOISSEAU P ; KIPARISSIDES C ; PAVESIO A ; SAXL O.** Nanotechnology for Health. Vision Paper and Basis for a Strategic Research Agenda for Nanomedicine. *European Technology Platform on Nanomedicine,* 2005
- **BOSSART J ; SEDO K ; KARARLI T.** a Decade in Review: Approved Products. *Delivery Report. Drug Delivery Products & Technologies,* 2000
- **CHEN Y ; HSIEH W ; LEE W ; ZENG D.** Effects of surface modification of PLGA-PEG-PLGA nanoparticles on loperamide delivery efficiency across the blood-brain barrier. *J Biomater Appl,* 2013, vol. 27 (7), 909-922
- **DZIADULEWICZ EK ; BEVAN SJ ; BRAIN CT ; COOTE PR ; CULSHAW AJ et al.** Naphthalen-1-yl-(4-pentyloxynaphthalen-1-yl)methanone: A potent, orally bioavailable human CB1/CB2 dual agonist with antihyperalgesic properties and restricted central nervous system penetration. *J. Med. Chem.,* 2007, vol. 50, 3851
- **GARCIA X ; ESCRIBANO E ; DOMENECH J ; QUERALT J ; FREIXES J.** In vitro characterization and in vivo analgesic and anti-allodynic activity of PLGA-bupivacaine nanoparticles. *Journal of Nanoparticle Research,* 2011, vol. 13 (5), 2213-2223
- **GARDIN A. ; KUCHER K ; KIESE B ; APPEL-DINGEMANSE S.** Cannabinoid receptor agonist 13, a novel cannabinoid agonist: First in human pharmacokinetics and safety. *Drug Metab. Dispos.,* 2009, vol. 37, 827
- **GORTH DJ ; MAUCK RL ; CHIARO JA ; MOHANRAJ B ; HEBELA NM ; DODGE GR ; ELLIOTT DM ; SMITH LJ.** IL-1ra delivered from poly(lactic-co-glycolic acid) microspheres attenuates IL-1β-mediated degradation of nucleus pulposus in vitro. *Arthritis Res Ther.,* 2012, vol. 3;14 (4), R179
- **HUDSON ; MARGARITIS A.** *Biopolymer nanoparticle production for controlled release of biopharmaceuticals,* June 2014, vol. 34 (2), 161-179
- **JAIN K ; GOYALA A ; MISHRA N ; VAIDYA B ; MANGAL S ; VYAS SP.** PEG-PLA-PEG block copolymeric nanoparticles for oral immunization against hepatitis B. *International Journal of Pharmaceutics,* 2010, vol. 387, 253-262
- **KIM S ; KIM JC ; SUL D ; HWANG SW ; LEE SH ; KIM YH et al.** Nanoparticle Formulation for Controlled Release of Capsaicin. *Journal of Nanoscience and Nanotechnology,* 2011, vol. 11 (5), 4586-4591

- **LAI P ; DAEAR W ; LÖBENBERG R ; PRENNER EJ.** Overview of the preparation of organic polymeric nanoparticles for drug delivery based on gelatine, chitosan, poly(d,l-lactide-co-glycolic acid) and polyalkylcyanoacrylate. *Colloids and Surfaces B: Biointerfaces,* 2014, vol. 118 (1), 154-163
- **LALANI J ; RATHI M ; LALAN M ; MISRA A.** Protein functionalized tramadol-loaded PLGA nanoparticles: preparation, optimization, stability and pharmacodynamic studies. *Drug Dev Ind Pharm,* 2013, vol. 39 (6), 854-864
- **LAM PL ; GAMBARI R.** Advanced progress of microencapsulation technologies: In vivo and in vitro models for studying oral and transdermal drug deliveries. *Journal of Controlled Release,* 2014, vol. 178, 25-45
- **MARTIN-BANDERAS L ; JOSEFA ALVAREZ-FUENTES ; MATILDE DURÁN-LOBATO ; JOSE PRADOS ; CONSOLACIÓN MELGUIZO ; MERCEDES FERNANDEZ-AREVALO ; MA ANGELES HOLGADO.** Cannabinoid derivate-loaded PLGA nanocarriers for oral administration: formulation, characterization, and cytotoxicity studies. *International Journal of Nanomedicine,* 2012, vol. 7, 5793-5806
- **MILLIGAN ED ; PENZKOVER KR ; SODERQUIST RG ; MAHONEY MJ.** Spinal Interleukin-10 Therapy to Treat Peripheral Neuropathic Pain. *Neuromodulation,* 2012, vol. 15 (6), 520-526
- **NOCERINO E ; AMATO M ; IZZOU AA.** Cannabis and cannabinoid receptor. *Fitoterapia,* 2000, vol. 71, 6-12
- **RATAJCZAK-ENSELME M ; ESTEBE J ; DOLLO G ; CHEVANNE F ; BEC D ; MALINOVSKY J et al.** Epidural, intrathecal and plasma pharmacokinetic study of epidural ropivacaine in PLGA-microspheres in sheep model. *European Journal of Pharmaceutics and Biopharmaceutics,* 2009, vol. 72 (1), 54-61
- **SHEN H ; HU X ; SZYMUSIAK M ; WANG ZJ ; LIU Y.** Orally Administered Nanocurcumin to Attenuate Morphine Tolerance: Comparison between Negatively Charged PLGA and Partially and Fully PEGylated Nanoparticles. *Molecular Pharmaceutics,* 2013, vol. 10 (12), 4546-4551
- **VILA A ; SANCHEZ A ; TOBÍO M ; CALVO P ; ALONSO MJ.** Design of biodegradable particles for protein delivery. *Journal of Controlled Release,* 2002, vol. 78, 15-24
- **WACKER M.** Nanocarriers for intravenous injection-The long hard road to the market. *International Journal of Pharmaceutics,* 2013, vol. 457 (1), 50-62
- **ZANELLA JM ; BURRIGHT EN ; HILDEBRAND K ; HOBOT C ; COX M ; CHRISTOFERSON L et al.** Effect of etanercept, a tumor necrosis factor-alpha inhibitor, on neuropathic pain in the rat chronic constriction injury model. *Spine 2008,* September 2014, vol. 33 (3), 227-234, http://www.marketsandmarkets.com/Market-Reports/drug-delivery-technologies-market-1085.html
- **OWENS DE ; PEPPAS NA.** Opsonization, biodistribution, and pharmacokinetics of polymeric nanoparticles. *International Journal of Pharmaceutics,* 2006, vol. 307 (1), 93-102